# FASCICULE DE BREVET EUROPEEN

(11) **EP 0 671 383 B1**
(45) Date de publication et mention de la délivrance du brevet: **07.01.1998**
(21) Numéro de dépôt: 95400481.8
(22) Date de dépôt: 06.03.1995
(51) Int. Cl.: C07C 233/36, C07C 233/38, D06M 13/405, C11D 1/52

(54) **Cire adoucissante pour les textiles, procédé pour sa préparation, compositions aqueuses la contenant et ses applications pour le traitement des textiles**
Wachsartiger Weichmacher für Textilien, Verfahren für seine Herstellung, ihn enthaltende wässrige Zusammensetzungen und seine Verwendung als Textilbehandlungsmittel
Softening wax for textiles, process for its preparation, aqueous composition containing it, and its use for the treatment of textiles

(30) Priorité: 08.03.1994 FR 9402654
(43) Date de publication de la demande: 13.09.1995
(73) Titulaire: SOCIETE FRANCAISE HOECHST, 92800 Puteaux (FR)
(72) Inventeur: Brian, Stéphane, F-75012 Paris (FR); Mercier, Michel, F-94410 Saint Maurice (FR); Blanc, Alain, F-93200 Saint Denis (FR)
(74) Mandataire: Santarelli, Marc

(56) Documents cités:
- EP-A- 0 188 242
- EP-A- 0 230 910
- EP-A- 0 248 365
- EP-A- 0 280 976
- DE-B- 1 048 412
- FR-A- 2 040 417

## Description

La présente invention concerne une cire adoucissante pour les textiles, un procédé pour sa préparation, des compositions aqueuses la contenant et ses applications pour le traitement des textiles.

Il est connu de traiter des fibres, des fils, des tricots, des tissus ainsi que des non-tissés à base de fibres cellulosiques et/ou de polyesters avec des compositions aqueuses afin de modifier ou d'améliorer leurs propriétés notamment leur toucher, leur tenue, leur glissement, leur douceur. Ces compositions peuvent être mises en oeuvre soit lors de l'apprêtage des textiles, soit directement dans des compositions détergentes, soit dans les bains de post-traitement des textiles.

Ces compositions sont notamment à base d'une hydroxyalkylpolyamine telle que la N-(hydroxy-2 éthyl) éthylènediamine et d'esters d'acides gras, et elles contiennent généralement une (hydroxy-2 éthyl)-1 Δ 1-imidazolidine substituée en position 1 par une chaîne grasse éventuellement salifiée par un acide minéral ou organique et/ou estérifiée sur la fonction alcool primaire de la chaîne latérale par un acide et/ou éthérifiée sur la fonction alcool primaire par une ou plusieurs molécules d'oxyde d'éthylène ou d'oxyde de propylène (demandes de brevet allemand N° 3 618 944, 3 135 235, 2 539 367, 2 539 335, 2 539 309, 2 210 087). Il est également connu d'employer comme adoucissant textile les produits de condensation d'un glycéride avec une hydroxyalkylpolyamine ainsi que divers dérivés de polyamines (cf par exemple l'état de la technique donné dans EP-A-0 345 842 ou dans EP-A-0 230 910). Afin d'améliorer la facilité du traitement des textiles par des produits adoucissants connus notamment en augmentant leur faculté de dilution et leur hydrophilie, de supprimer les jaunissements parasites des textiles traités par ces produits adoucissants, et enfin d'augmenter la stabilité des compositions aqueuses les contenant, la demanderesse a découvert avec étonnement une cire adoucissante obviant aux inconvénients des produits connus et améliorant le toucher des textiles traités par des compositions aqueuses les contenant.

La présente invention a donc pour objet une cire qui peut être préparée par mise en réaction de tristéarine, et de N-(hydroxy-2 éthyl) éthylènediamine suivie de la salification par un acide organique des fonctions amines non transformées caractérisée par le fait que
(i) la réaction est effectuée en présence de polyéthylèneglycol 1000, à une température de 140 ± 5°C, en présence d'un catalyseur basique jusqu'à ce que 80 ± 15 % des fonctions amines aient été transformées et
(ii) qu'ensuite le milieu réactionnel est acidifié avec de l'acide acétique avant d'être refroidi à la température ambiante.

Par "catalyseur basique", on désigne des bases organiques ou inorganiques telles que les hydroxydes de métal alcalin, les alcanolates en C₁-C₄ de métal alcalin ou les hydroxydes d'ammoniums quaternaires. Avantageusement, le catalyseur basique est le méthanolate de sodium ou la potasse éthanolique. On utilise des quantités de base de préférence inférieures ou égales à 8 moles et notamment environ 5 moles et moins pour 100 moles de stéarine mise en oeuvre.

L'acidification du milieu réactionnel par l'acide acétique est avantageusement réalisée dans la masse fondue de la cire préalablement refroidie vers 90°C avec une solution aqueuse contenant pondéralement plus de 50 % d'acide acétique.

Le procédé ci-dessus est de préférence entièrement réalisé en atmosphère inerte, avantageusement sous azote, pour éviter toute coloration parasite.

La cire telle qu'elle peut être obtenue par le procédé ci-dessus décrit se présente à la température ambiante sous la forme d'un solide blanc, présentant une bonne hydrophilie et se liquéfiant vers 90°C environ.

Dans des conditions avantageuses de mise en oeuvre, le procédé ci-dessus décrit permettant la préparation des cires selon l'invention est réalisé de la manière suivante :
- pour une mole de tristéarine on utilise 1,025 ± 0,025 mole de N-(hydroxy-2 éthyl) éthylènediamine et 0,375 ± 0,025 mole de polyéthylèneglycol 1000.

La disparition des fonctions amines dans le milieu réactionnel est suivie par des moyens connus en soi. Avantageusement, on prélève régulièrement un échantillon du milieu réactionnel, de poids connu, que l'on soumet à un dosage potentiométrique en milieu non-aqueux avec une solution d'acide chlorhydrique dans un alcanol, de titre connu. Dans des conditions particulièrement avantageuses, on effectue ce dosage à 60°C, dans une solution éthylèneglycol / propanol-2, 1/1 en volume contenant environ 5 % en poids de l'échantillon, avec une solution d'acide chlorhydrique 0,1 M dans le propanol-2, en utilisant un titroprocessor METROHM équipé d'une électrode de verre combiné.

L'invention a aussi pour objet un procédé de préparation d'une cire ci-dessus décrite caractérisé en ce que
i) l'on fait réagir de la tristéarine et de la N-(hydroxy-2 éthyl) éthylènediamine en présence de polyéthylèneglycol 1000, à une température de 140 ± 5°C, en présence d'un catalyseur basique jusqu'à ce que 80 ± 15 % des fonctions amines aient été transformées et
(ii) qu'ensuite le milieu réactionnel est acidifié avec de l'acide acétique avant d'être refroidi à la température ambiante.

L'invention a également pour objet une composition aqueuse adoucissante, destinée aux traitements des textiles, caractérisée par le fait qu'elle contient, en dispersion dans l'eau, une cire obtenue par le procédé ci-dessus décrit. Avantageusement, la composition selon l'invention contient pondéralement 20 ± 5 % de cire de l'invention.

Selon l'invention, ces compositions aqueuses adoucissantes peuvent être obtenues par un procédé caractérisé par le fait que l'on disperse dans de l'eau, à une température d'environ 40°C, sous forte agitation et en atmosphère inerte, la quantité désirée de cire pouvant être obtenue comme indiqué ci-dessus, préalablement liquéfiée à une température d'environ 90°C, puis que l'on refroidit à la température ambiante la dispersion obtenue.

On obtient ainsi une composition selon l'invention présentant une viscosité Brookfield déterminée à 20°C avec un viscosimètre Brookfield RVT équipé de l'axe 1, tournant à la vitesse de 50 tr/mn, inférieure ou égale à 180 mPa.s.

Les compositions aqueuses de l'invention telles que définies ci-dessus présentent d'intéressantes propriétés adoucissantes des textiles. Elles communiquent aux textiles, traités en milieux aqueux selon les techniques habituelles, un toucher agréable sans occasionner un jaunissement. De plus, elles sont stables dans le temps, aisément diluables à l'eau et hydrophiles.

Par "textile", on désigne les fibres, les fils, les tissus, les tricots, les non-tissés à base de coton, de polyester, de matière acrylique, de lin, de polyamide, de viscose et leurs associations ou mélanges.

Par "stabilité dans le temps", on désigne l'absence totale de sédimentation d'une solution aqueuse contenant 2 g/l, exprimé en produit sec de la composition de l'invention, après un repos de 7 jours à 20°C.

Les compositions aqueuses selon l'invention présentent un pH d'environ 5 et elles peuvent également contenir divers additifs classiques tels que conservateur(s), arôme(s), colorant(s), accélérateur de dispersion.

Les compositions de l'invention sont facilement applicables en dispersion aqueuse sur les textiles par les techniques usuelles telles que les procédé d'enduction, d'impression-essorage, de foulardage ou de pulvérisation. Elles peuvent également être appliquées aux textiles au cours des opérations classiques d'ennoblissement des textiles avec des résines aminoplastes à base d'urée telles que les résines dérivées de la dihydroxyéthylèneurée.

L'utilisation des compositions selon l'invention pour le traitement adoucissant des textiles constitue un autre objet de l'invention. Les doses d'emploi peuvent varier dans de grandes proportions selon la nature de l'article à traiter et le but recherché. Habituellement on peut utiliser de 20 à 50 g/l de la composition selon l'invention.

Les exemples suivants illustrent la présente invention sans toutefois la limiter.

### EXEMPLE 1

Dans 375 g (0,375 mole) de polyéthylèneglycol 1000 maintenu à 50°C environ, on introduit sous agitation et en atmosphère inerte successivement :
- 891,51 (1 mole) de tristéarine,
- 107,3 g (1,03 mole) de N-(hydroxy-2 éthyl) éthylènediamine,
   puis on chauffe le milieu réactionnel sous agitation en le maintenant en atmosphère inerte. Vers 90°C, lorsque le milieu devient liquide, on introduit :
- 2,7 g (50 mmoles) de méthanolate de sodium en solution à 30 % en poids dans du méthanol.

On poursuit ensuite le chauffage de manière à élever la température du milieu réactionnel à 140°C et on l'abandonne à cette température, sous agitation et en atmosphère inerte, jusqu'à disparition de 85 % environ des fonctions amines. Pour ce faire, on prélève régulièrement dans le milieu réactionnel un échantillon que l'on dose par potentiométrie, à 60°C, en solution à 5 % en poids dans un mélange éthylèneglycol / propanol-2, 1/1 en volume avec une solution d'acide chlorhydrique 0,1 M dans le propanol-2. A ce stade, on refroidit le milieu réactionnel à 90°C environ et, à cette température, on introduit toujours sous agitation et en atmosphère inerte :
- 33,4 (0,55 mole) d'acide acétique en solution aqueuse à 80 % en poids.

Un échantillon de cette cire est refroidi à la température ambiante puis analysé par RMN du proton et du carbone treize. On note l'absence d'heptadécyl-2 (hydroxy-2 éthyl)-1 Δ 2-imidazoline.

Après acidification, le milieu réactionnel maintenu à 90°C environ est versé sous forte agitation dans 6280 g d'eau en maintenant la température inférieure à 40°C. La dispersion ainsi obtenue est ensuite chauffée à 90°C puis elle est abandonnée 30 minutes à cette température avant d'être refroidie à la température ambiante. On obtient ainsi une dispersion aqueuse blanche, présentant un pH de 5, une viscosité Brookfield déterminée à 20°C (axe 1, vitesse 50) avec un viscosimètre Brookfield RVT de 150 mPa.s et un extrait sec de 17,5 % déterminé par chauffage de 3 heures à 105°C.

En dispersant sous agitation, à la température ambiante, 1143 g de cette composition aqueuse dans 100 litres d'eau bipermutée, on obtient un bain aqueux contenant environ 2 g/l d'extraits secs (déterminés par chauffage d'un échantillon durant 3 heures à 105°C). Ce bain abandonné 7 jours à la température ambiante ne présente ni sédimentation, ni relarguage, il est parfaitement stable.

## Revendications

1. Cire adoucissante pour le traitement des textiles, qui peut être préparée par mise en réaction de tristéarine, et de N-(hydroxy-2 éthyl) éthylènediamine, suivie de la salification par un acide organique des fonctions amines non transformées, caractérisée par le fait que
(i) la réaction est effectuée en présence de polyéthylèneglycol 1000, à une température de 140 ± 5°C, en présence d'un catalyseur basique jusqu'à ce que 80 ± 15 % des fonctions amines aient été transformées et
(ii) qu'ensuite le milieu réactionnel est acidifié avec de l'acide acétique avant d'être refroidi à la température ambiante.

2. Cire selon la revendication 1, caractérisée par le fait que l'on met en oeuvre pour 1 mole de tristéarine, 1,025 ± 0,025 mole de N-(hydroxy-2 éthyl) éthylènediamine et 0,375 ± 0,025 mole de polyéthylèneglycol 1000.

3. Procédé de préparation d'une cire selon la revendication 1, caractérisé en ce que
i) l'on fait réagir de la tristéarine et de la N-(hydroxy-2 éthyl) éthylènediamine en présence de polyéthylèneglycol 1000, à une température de 140 ± 5°C, en présence d'un catalyseur basique jusqu'à ce que 80 ± 15 % des fonctions amines aient été transformées et
(ii) qu'ensuite le milieu réactionnel est acidifié avec de l'acide acétique avant d'être refroidi à la température ambiante.

4. Compositions aqueuses adoucissantes pour le traitement des textiles, caractérisées par le fait qu'elles contiennent en dispersion dans de l'eau une cire selon l'une quelconque des revendications 1 et 2.

5. Compositions aqueuses adoucissantes pour le traitement des textiles selon la revendication 4, caractérisées par le fait qu'elles contiennent pondéralement 20 ± 5 % de cire.

6. Procédé pour obtenir une composition aqueuse adoucissante pour le traitement des textiles selon l'une quelconque des revendications 4 et 5, caractérisé par le fait que l'on disperse sous forte agitation dans de l'eau, à une température d'environ 40°C, la quantité désirée de cire telle que définie dans l'une quelconque des revendications 1 et 2, préalablement liquéfiée à une température d'environ 90°C, puis que l'on refroidit à la température ambiante la dispersion obtenue.

7. Application d'une composition aqueuse selon l'une quelconque des revendications 4 et 5 dans un traitement adoucissant des textiles.

## Claims

1. Softening wax for the treatment of textiles, which can be prepared by reacting tristearin and N-(2-hydroxy ethyl) ethylenediamine, followed by salification by an organic acid of the non-converted amine functions, characterized by the fact that
(i) the reaction is carried out in the presence of polyethyleneglycol 1000, at a temperature of 140 ± 5°C, in the presence of a basic catalyst until 80 ± 15% of the amine functions have been converted and
(ii) that then the reaction medium is acidified with acetic acid before being cooled down to ambient temperature.

2. Wax according to claim 1, characterized by the fact that 1.025 ± 0.025 mole of N-(2-hydroxy ethyl) ethylenediamine and 0.375 ± 0.025 mole of polyethyleneglycol 1000 are used per 1 mole of tristearin.

3. Preparation process for a wax according to claim 1, characterized in that
(i) tristearin and N-(2-hydroxy ethyl) ethylenediamine are reacted in the presence of polyethyleneglycol 1000, at a temperature of 140 ± 5°C, in the presence of a basic catalyst until 80 ± 15% of the amine functions have been converted and
(ii) that then the reaction medium is acidified with acetic acid before being cooled down to ambient temperature.

4. Aqueous softening compositions for the treatment of textiles, characterized by the fact that they contain in dispersion in water a wax according to any one of claims 1 and 2.

5. Aqueous softening compositions for the treatment of textiles according to claim 4, characterized by the fact that they contain 20 ± 5% by weight of wax.

6. Process for obtaining an aqueous softening composition for the treatment of textiles according to any one of claims 4 and 5, characterized by the fact that the desired quantity of wax as defined in any one of claims 1 and 2, liquefied beforehand at a temperature of about 90°C, is dispersed under vigorous agitation in water, at a temperature of about 40°C, then the dispersion obtained is cooled down to ambient temperature.

7. Use of an aqueous composition according to any one of claims 4 and 5 in a textile softening treatment.

## Patentansprüche

1. Weichmachendes Wachs zur Behandlung von Textilien, dadurch erhältlich, daß man Tristearin und N-(2-Hydroxyethyl)-ethylendiamin umsetzt, gefolgt von Salzbildung mittels einer organischen Säure an den nicht-umgesetzten Aminfunktionen, dadurch gekennzeichnet, daß
(i) die Reaktion in Gegenwart von Polyethylenglycol 1000 bei einer Temperatur von 140 ± 5°C in Gegenwart eines basischen Katalysators durchgeführt wird, bis 80 ± 50% der Aminfunktionen umgesetzt sind, und
(ii) dann das Reaktionsmedium mit Essigsäure angesäuert wird, bevor es auf Umgebungstemperatur abgekühlt wird.

2. Wachs nach Anspruch 1, dadurch gekennzeichnet, daß man auf 1 Mol Tristearin 1,025 ± 0,025 Mol N-(2-Hydroxyethyl)-ethylendiamin und 0,375 ± 0,025 Mol Polyethylenglycol 1000 verwendet.

3. Verfahren zur Herstellung eines Wachses nach Anspruch 1, dadurch gekennzeichnet, daß
(i) man das Tristearin und N-(2-Hydroxyethyl)-ethylendiamin in Gegenwart von Polyethylenglycol 1000 bei einer Temperatur von 140 ± 5°C in Gegenwart eines basischen Katalysators umsetzt, bis 80 ± 15% der Aminfunktionen umgesetzt worden sind, und
(ii) dann das Reaktionsmedium mit Essigsäure ansäuert, bevor es auf Umgebungstemperatur abgekühlt wird.

4. Wäßrige Weichmacherzusammensetzungen zur Behandlung von Textilien, dadurch gekennzeichnet, daß sie in Dispersion in Wasser ein Wachs nach einem der Ansprüche 1 und 2 enthalten.

5. Wäßrige Weichmacherzusammensetzungen zur Behandlung von Textilien nach Anspruch 4, dadurch gekennzeichnet, daß sie 20 ± 5 Gew.-% Wachs enthalten.

6. Verfahren zur Erzielung einer wäßrigen Weichmacherzusammensetzung zur Behandlung von Textilien nach einem der Ansprüche 4 und 5, dadurch gekennzeichnet, daß man unter starkem Rühren in Wasser bei einer Temperatur von etwa 40°C die gewünschte Menge des Wachses nach Definition in einem der Ansprüche 1 und 2, das vorher bei einer Temperatur von etwa 90°C verflüssigt wurde, dispergiert, worauf man die erhaltene Dispersion auf Umgebungstemperatur abkühlt.

7. Verwendung einer wäßrigen Zusammensetzung nach einem der Ansprüche 4 und 5 bei der Weichmachbehandlung von Textilien.
